(19) **Europäisches Patentamt / European Patent Office / Office européen des brevets**

(11) **EP 4 721 675 A1**

(12) **EUROPEAN PATENT APPLICATION**
published in accordance with Art. 153(4) EPC

(43) Date of publication:
**08.04.2026 Bulletin 2026/15**

(21) Application number: **24814480.0**

(22) Date of filing: **29.05.2024**

(51) International Patent Classification (IPC):
***A61B 8/06*** (2006.01)    ***A61B 8/00*** (2006.01)

(86) International application number:
**PCT/CN2024/096056**

(87) International publication number:
**WO 2024/245283 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.05.2023 CN 202310620741**

(71) Applicant: **Wuxi Hisky Medical Technologies Co., Ltd.**
**Wuxi, Jiangsu 214000 (CN)**

(72) Inventors:
• **HE, Qiong**
  **Wuxi, Jiangsu 214000 (CN)**
• **XU, Xiaochen**
  **Wuxi, Jiangsu 214000 (CN)**
• **SHAO, Jinhua**
  **Wuxi, Jiangsu 214000 (CN)**
• **SUN, Jin**
  **Wuxi, Jiangsu 214000 (CN)**

(74) Representative: **Elzaburu S.L.P.**
**Paseo de la Castellana 259C**
**Torre de Cristal, planta 28**
**28046 Madrid (ES)**

(54) **BLOOD SUPPLY INFORMATION EXTRACTION METHOD AND APPARATUS, AND DEVICE AND READABLE STORAGE MEDIUM**

(57)    The present application discloses a blood supply information extraction method and apparatus, an electronic device, and a computer-readable storage medium, where the blood supply information extraction method includes: controlling an ultrasonic probe to emit ultrasonic waves to a target body from a plurality of angles, and obtaining ultrasonic echo data of the target body at the plurality of angles, where the imaging areas of the ultrasonic waves emitted from the plurality of angles in the target body have an overlapping area; for the ultrasonic echo data at each angle, respectively performing a blood supply information extraction to obtain blood supply information of the target body at each angle; obtaining blood supply information of the target body in the overlapping area based on the blood supply information at each angle. The ultrasonic probe is controlled to emit ultrasonic waves to the target body from a plurality of different angles, and the imaging areas of the ultrasonic waves from the different angles in the target body have the overlapping area. Based on the ultrasonic echo data from a plurality of different angles, more accurate analysis can be performed on the blood supply information in the overlapping area, thus, the blood supply information with higher accuracy can be obtained.

Controlling an ultrasonic probe to emit ultrasonic waves to a target body from a plurality of angles, and obtaining ultrasonic echo data of the target body at the plurality of angles — S21

For the ultrasonic echo data at each angle, respectively performing a blood supply information extraction to obtain blood supply information of the target body at each angle — S22

Obtaining blood supply information of the target body in the overlapping area based on the blood supply information at each angle — S23

FIG. 2

## Description

The present application claims priority to Chinese Patent Application No. 202310620741.9, filed with the China National Intellectual Property Administration on May 30, 2023 and entitled "BLOOD SUPPLY INFORMATION EXTRACTION METHOD AND APPARATUS, DEVICE, AND READABLE STORAGE MEDIUM", which is incorporated herein by reference in its entirety.

## TECHNICAL FIELD

The present application relates to the field of biomedicine and, in particular, to a blood supply information extraction method and apparatus, a device, and a readable storage medium.

## BACKGROUND

Ultrasonic imaging is widely used in clinical diagnosis due to its advantages of rapidity, non-invasiveness, real-time performance, and no ionizing radiation. With the development of medical technologies, ultrasound can not only achieve structural imaging but also perform functional imaging. For example, the ultrasonic blood flow imaging technology is an important branch in the field of medical ultrasonic imaging, which can non-destructively extract blood supply information of the human body and obtain hemodynamic parameters, and is a major breakthrough in ultrasound diagnostic technologies. By utilizing the reflected signals of ultrasound in tissues and extracting tissue blood supply information through algorithms, the tissue blood flow can be imaged non-destructively, without the need for contrast agents.

However, at present, the accuracy of the extracted tissue blood supply information still needs to be improved.

## SUMMARY

In view of this, embodiments of the present application provide a blood supply information extraction method and apparatus, an electronic device, and a computer-readable storage medium, the accuracy of the obtained blood supply information is higher.

An aspect of the present application provides a blood supply information extraction method, where the method includes:

controlling an ultrasonic probe to emit ultrasonic waves to a target body from a plurality of angles, and obtaining ultrasonic echo data of the target body at the plurality of angles, where the ultrasonic wave emitted from each angle has a corresponding imaging area in the target body, and the imaging areas of the ultrasonic waves emitted from the plurality of angles in the target body have an overlapping area;
for the ultrasonic echo data at each angle, respectively performing a blood supply information extraction to obtain blood supply information of the target body at the each angle; and
obtaining blood supply information of the target body in the overlapping area based on the blood supply information at the each angle.

In some embodiments, a beam width of at least some of the ultrasonic waves emitted from the plurality of angles is not less than 3 ultrasonic wavelengths.

In some embodiments, where the ultrasonic probe includes an array element set, and the array element set includes a plurality of array elements;
the controlling the ultrasonic probe to emit ultrasonic waves to the target body from the plurality of angles includes:

determining an ultrasonic emission sequence corresponding to each angle, where the ultrasonic emission sequence is used to characterize a time interval between emissions of the ultrasonic waves by each array element in the array element set;
controlling each array element in the array element set to emit the ultrasonic wave to the target body according to the ultrasonic emission sequence corresponding to the each angle.

In some embodiments, where the controlling each array element in the array element set to emit the ultrasonic wave to the target body according to the ultrasonic emission sequence corresponding to the each angle includes:
controlling the array elements in the array element set to emit the ultrasonic waves to the target body according to the ultrasonic emission sequence corresponding to the each angle at intervals of a preset time period.

In some embodiments, where the ultrasonic probe includes a plurality of array element sets, each of the array element sets includes a plurality of array elements;
the controlling the ultrasonic probe to emit ultrasonic waves to the target body from the plurality of angles includes:

determining an ultrasonic emission sequence corresponding to the each angle, where the ultrasonic emission sequence is used to characterize a time interval between emissions of the ultrasonic waves by each array element in the array element set;
sequentially controlling the array elements of each array element set to emit the ultrasonic waves to the target body according to the ultrasonic emission sequences corresponding to the respective angles based on an angle emission timing sequence of the plurality of angles.

In some embodiments, where for the ultrasonic echo data at each angle, the respectively performing the blood supply information extraction to obtain the blood supply information of the target body at the each angle includes:

performing decomposition on the ultrasonic echo data at the each angle based on a principal component decomposition method to obtain the blood supply information at the each angle.

In some embodiments, where the performing decomposition on the ultrasonic echo data at the each angle based on the principal component decomposition method to obtain the blood supply information at the each angle includes:

storing the ultrasonic echo data at the each angle in different matrices respectively to obtain an echo data matrix of the each angle, and during the decomposition, performing at least one row-column transposition operation on the ultrasonic echo data in the echo data matrix of the each angle to decompose and obtain the blood supply information at the each angle from each ultrasonic echo data after performing the row-column transposition operation.

In some embodiments, where the performing decomposition on the ultrasonic echo data at the each angle includes:

performing decomposition on the ultrasonic echo data at the each angle using a parallel decomposition manner.

In some embodiments, where the performing decomposition on the ultrasonic echo data at the each angle based on the principal component decomposition method to obtain the blood supply information at the each angle includes:

performing parallel decomposition on the ultrasonic echo data at the each angle using a graphics processor based on a principal component decomposition method to obtain the blood supply information at the each angle.

A further aspect of the present application provides a blood supply information extraction apparatus, where the apparatus includes:

a control module, configured to control an ultrasonic probe to emit ultrasonic waves to a target body from a plurality of angles, and obtain ultrasonic echo data of the target body at the plurality of angles, where the ultrasonic wave emitted from each angle has a corresponding imaging area in the target body, and the imaging areas of the ultrasonic waves emitted from the plurality of angles in the target body have an overlapping area;
an extraction module, configured to, for the ultrasonic echo data at each angle, respectively perform a blood supply information extraction to obtain blood supply information of the target body at the each angle;
a determination module, configured to obtain blood supply information of the target body in the overlapping area based on the blood supply information at the each angle.

Another aspect of the present application also provides a computer-readable storage medium, where the computer-readable storage medium is used to store a computer program, which, when executed by a processor, implements the method as described above.

Yet another aspect of the present application also provides an electronic device, where the electronic device includes a processor and a memory, the memory is configured to store a computer program, which, when executed by the processor, implements the method as described above.

Still another aspect of the present application also provides a chip, where the chip includes a memory and a processor, the memory stores code and data, the memory is coupled to the processor, and the processor runs a program in the memory to enable the chip to perform the method as described above.

Still another aspect of the present application also provides a program product including a computer program, which, when the program product runs on a computer, enables the computer to perform the method as described above.

Still another aspect of the present application also provides a computer program, which, when executed by a processor, is configured to perform the method as described above.

In technical solutions of some embodiments of the present application, the ultrasonic probe is controlled to emit ultrasonic waves to the target body from a plurality of different angles, and the imaging areas of the ultrasonic waves from the different angles in the target body have the overlapping area. In this way, there are ultrasound echo data at a plurality of angles in the overlapping area. Based on the ultrasonic echo data from a plurality of different angles, more accurate analysis can be performed on the blood supply information in the overlapping area, thereby, greatly improving the accuracy of the obtained blood supply information.

**BRIEF DESCRIPTION OF DRAWINGS**

By referring to the accompanying drawings, the features and advantages of the present application will be more clearly understood. The accompanying drawings are illustrative and should not to be taken as limiting the present application in any way. In the accompanying drawings:

FIG. 1 shows a schematic diagram of ultrasonic detection when extracting blood supply information in some technologies;
FIG. 2 shows a schematic flowchart of a blood supply information extraction method provided by an embodiment of the present application;
FIG. 3 shows a schematic diagram of an ultrasonic probe emitting ultrasonic waves provided by another embodiment of the present application;
FIG. 4 shows a schematic diagram of an ultrasonic probe emitting ultrasonic waves provided by another embodiment of the present application;

FIG. 5 shows a schematic diagram of an ultrasonic probe emitting ultrasonic waves provided by still another embodiment of the present application;

FIG. 6 shows a schematic diagram of ultrasonic detection by some blood supply information extraction scenarios;

FIG. 7 shows a schematic diagram of a blood flow direction extraction provided by some embodiments of the present application;

FIG. 8 shows a schematic flowchart of principal component decomposition provided by an embodiment of the present application;

FIG. 9 shows a schematic diagram of functional modules of a blood supply information extraction apparatus provided by an embodiment of the present application;

FIG. 10 shows a schematic structural diagram of an electronic device provided by an embodiment of the present application.

## DESCRIPTION OF EMBODIMENTS

In order to make the purpose, technical solution, and advantages of the implementations of the present application clearer, the following will provide a clear and complete description of the technical solutions in the implementations of the present application in conjunction with the accompanying drawings in the implementations of the present application. Obviously, the described implementations are a part of the implementations of the present application, not all of them. Based on the implementations described in the present application, all other implementations obtained by those skilled in the art without creative labor are within the protection scope of the present application.

The blood supply information includes but is not limited to a blood flow velocity magnitude, a blood flow velocity gradient, an arterial-venous blood flow velocity ratio, and blood vessel distribution information. The blood vessel distribution information can further include blood vessel density, blood vessel diameter, blood vessel tortuosity, etc.

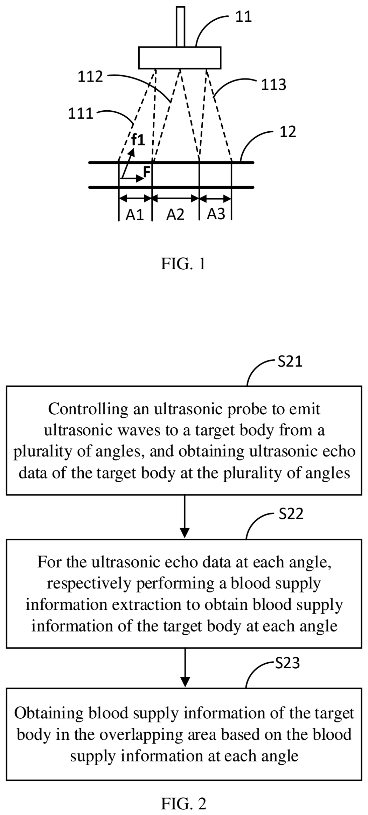

Please refer to FIG. 1, which is a schematic diagram of ultrasonic detection when extracting blood supply information in some technologies. In FIG. 1, when extracting the blood supply information of the tissue 12, the ultrasonic probe 11 can sequentially emit a plurality of narrow-beam ultrasonic waves towards the tissue 12. Each narrow-beam ultrasonic wave has a different imaging area in the tissue 12. Based on the ultrasonic echoes of various narrow-beam ultrasonic waves, the blood supply information of the tissue 12 in different imaging areas can be extracted. The blood supply information of the tissue 12 in various imaging areas is integrated, so as to obtain the complete blood supply information of tissue 12.

For example, in FIG. 1, assuming that the blood supply information of the tissue 12 needs to be extracted, in sequence, firstly, the ultrasonic probe 11 is controlled to emit the narrow-beam ultrasonic wave 111, and the blood supply information of the imaging area A1 can be extracted based on the ultrasonic echo of the narrow-beam ultrasonic wave 111; then, the ultrasonic probe 11 is controlled to emit the narrow-beam ultrasonic wave 112, and the blood supply information of the imaging area A2 can be extracted based on the ultrasonic echo of the narrow-beam ultrasonic wave 112; finally, the ultrasonic probe 11 is controlled to emit the narrow-beam ultrasonic wave 113, and the blood supply information of the imaging area A3 can be extracted based on the ultrasonic echo of the narrow-beam ultrasonic wave 113. The blood supply information of the imaging areas A1, A2, and A3 is integrated to obtain the blood supply information of the tissue 12.

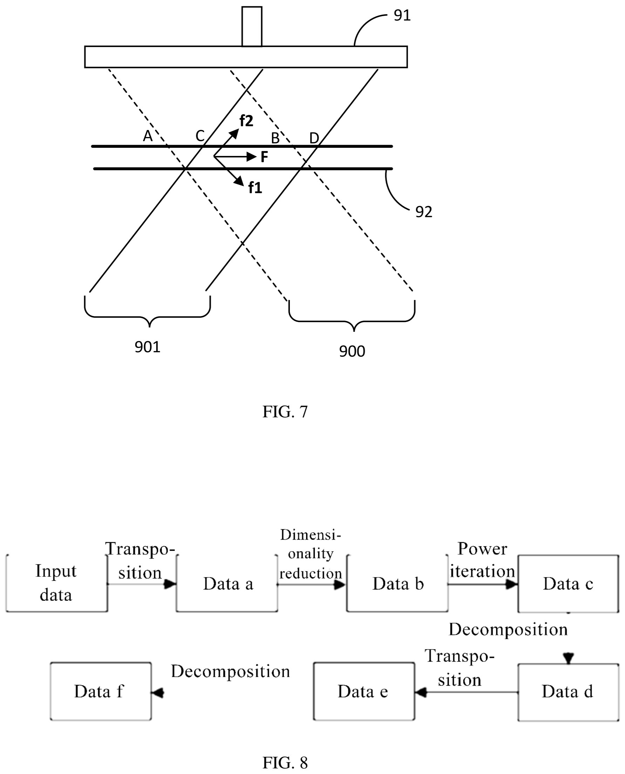

The blood supply information obtained in this extracting method for the blood supply information is in low accuracy and slow speed. Take the extraction of the blood flow direction as an example. In the method shown in FIG. 1, referring to FIG. 7, only a direction component f1 or f2 of the blood flow direction F in the ultrasonic wave direction can be extracted. The accurate direction of the blood flow cannot be obtained based on the extracted direction component f1 or f2, therefore the accuracy of the extracted blood supply information is not high.

In view of this, the present application provides a blood supply information extraction method, by which the obtained blood supply information has higher accuracy. The blood supply information extraction method can be applied to an electronic device. The electronic device includes but is not limited to a medical device. Please refer to FIG. 2, which is a schematic flowchart of a blood supply information extraction method provided by an embodiment of the present application. In FIG. 2, the blood supply information extraction method may include the following steps:

Step S21, controlling an ultrasonic probe to emit ultrasonic waves to a target body from a plurality of angles, and obtaining ultrasonic echo data of the target body at the plurality of angles, where the ultrasonic wave emitted from each angle has a corresponding imaging area in the target body, and the imaging areas of the ultrasonic waves emitted from the plurality of angles in the target body have an overlapping area.

In some embodiments, the target body is the tissue for which blood supply information needs to be extracted, such as breast tissue in the human body or tumor tissue in the breast. The ultrasonic echo data is data extracted from a reflected ultrasound wave after an ultrasonic wave sent by the ultrasonic probe to the target body is reflected. The blood supply information of the target body at a corresponding angle can be analyzed based on the ultrasonic echo data at each angle.

Where a plurality of angles are different, that is, the ultrasonic waves are emitted to the target body from a plurality of different angles.

In some embodiments, the direction in which the ultrasonic probe is perpendicular to the skin surface corresponding to the target body at the detection position is taken as 0 degree direction, and a plurality of angles are deflection angles relative to the 0 degree direction. It should be noted that the deflection angle can be 0 degree.

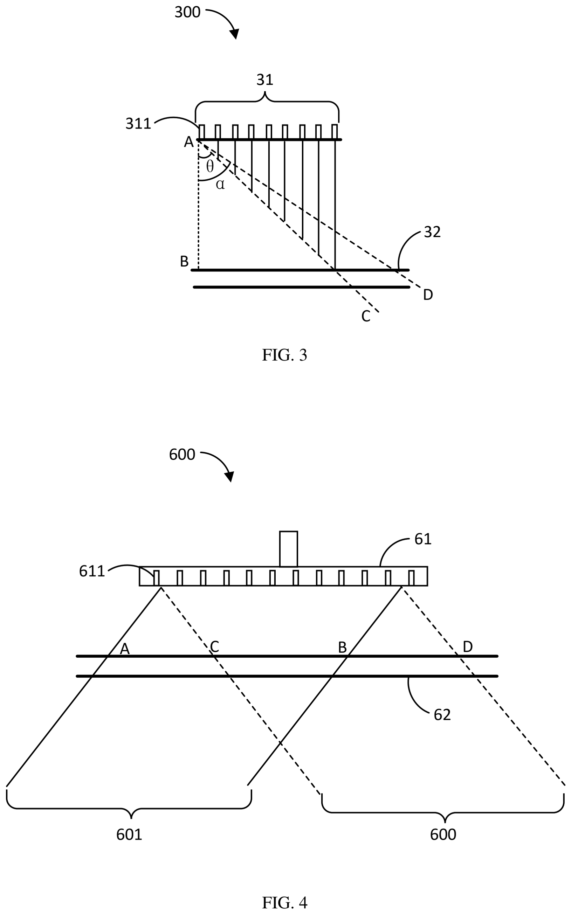

For ease of understanding, please refer to FIG. 3, which is a schematic diagram of an ultrasonic probe 300 emitting ultrasonic waves provided by an embodiment of the present application. In FIG. 3, assuming that the ultrasonic wave is emitted towards the target body 32 along the AC direction, the ultrasonic wave along the AC direction is deflected to the right by θ degrees relative to the 0 degree direction (i.e. AB direction). Therefore, it can be said that the ultrasonic wave in the AC direction is the ultrasonic wave emitted by the ultrasonic probe 300 from the angle θ towards the target body 32.

Continuing to refer to FIG. 3, in some embodiments, the ultrasonic probe 300 may include an array element set 31, and the array element set 31 may include a plurality of array elements 311 used to emit an ultrasonic wave and receive an ultrasonic echo. Where the number of array elements 311 included in the array element set 31 can be set according to the requirements. Each array element 311 in the array element set 31 can emit the ultrasonic wave along the 0 degree direction at the detection position. Each array element 311 of the ultrasonic probe 300 is controlled to emit the ultrasonic wave at a certain time interval, so as to enable the ultrasonic probe 300 to emit the ultrasonic waves at an inclined angle at the detection position, thereby achieving the emission of ultrasonic waves from different angles towards the target body 32.

For example, assuming that it is needed for the ultrasonic probe 300 to emit the ultrasonic wave from the angle θ to the target body 32, each array element 311 can be controlled in sequence from right to left to emit the ultrasonic wave at intervals of t. That is, the 1st array element 311 is controlled to emit the ultrasonic wave starting from time T, the 2nd array element 311 is controlled to emit the ultrasonic wave starting from time T+t, the 3rd array element 311 is controlled to emit the ultrasonic wave starting from time T+t+t, and so on. Due to the time interval between the emissions of the ultrasonic waves by each array element 311, the ultrasonic wave along the AC direction, i.e., the ultrasonic wave that can be emitted to the target body 32 from the angle θ, can be obtained.

It can be understood that by controlling the time interval for emitting ultrasonic waves by each array element, the ultrasonic probe can emit the ultrasonic waves from different angles to the target body. For example, assuming that it is needed to control the ultrasonic probe 300 to emit the ultrasonic wave from the angle θ to the target body 32, then each array element 311 can be controlled in sequence from right to left to start emitting the ultrasound wave at intervals of t milliseconds; assuming that it is needed to control the ultrasonic probe 300 to emit the ultrasonic wave from the angle α to the target body 32, then each array element 311 can be controlled in sequence from right to left to start emitting the ultrasound wave at intervals of t1 milliseconds. Where the values of t and t1 are different. In other words, each angle has a corresponding ultrasonic emission sequence, where the ultrasonic emission sequence is used to characterize a time interval between the emissions of the ultrasonic waves by each array element 311 in the array element set. When transmitting the ultrasonic wave, firstly, based on an angle of an ultrasonic wave to be emitted (which can also be understood as based on an angle at which the ultrasonic wave is intended to be emitted to the target body), the ultrasonic emission sequence corresponding to that angle can be determined. Then, each array element 311 in the array element set 31 is controlled to emit the ultrasonic wave to the target body 32 according to the ultrasonic emission sequence, thereby achieving the emission of the ultrasonic wave from the above angle to the target body.

In the actual detection, the ultrasonic probe can be provided with one or more array element sets according to actual needs. The following will be explained separately according to the number of array element sets.

Please refer to FIG. 4, which is a schematic diagram of an ultrasonic probe 600 emitting ultrasonic waves provided by an embodiment of the present application. In FIG. 4, the ultrasonic probe 600 includes one array element set 61. When the ultrasonic probe 600 needs to emit ultrasonic waves to the target body 62 from a plurality of angles, the ultrasonic emission sequence corresponding to each angle can be firstly determined, and each array element 611 in the array element set 61 is controlled to emit the ultrasonic wave to the target body 62 according to the ultrasonic emission sequence corresponding to each angle, thereby achieving the emission of the ultrasonic wave from a plurality of angles to the target body.

In order to obtain more accurate ultrasonic echo data at each angle and avoid mutual interference between ultrasonic waves emitted from each angle, optionally, the ultrasonic probe 600 can control the array elements 611 in the array element set 61 to emit the ultrasonic waves towards the target body according to the ultrasonic emission sequence corresponding to each angle at intervals of a preset time period. Simply put, at intervals of every preset time period, the ultrasonic probe 600 emits the ultrasonic waves from one of the angles to the target body 62. In this way, the emission of the ultrasound waves from N angles is completed at intervals of N-1 preset time periods, where N is the number of angles. The duration of the preset time period can be set according to needs, such as 1ms, 3ms, etc. It can also be calculated according to a target depth at which the ultrasonic wave propagates within the target body, for example, calculated through formula 2d/c, where d is target depth at which the ultrasonic wave propagates within the target body, the target depth of different target bodies may be different; c is a propagation speed of the ultrasonic wave in the target body, of course, the preset time period can also be determined through other methods, which is not limited in this application. The target depth is related to the depth of the area of interest within the target body, and the specific depth can be set according to the needs.

For example, assuming that the preset time interval is 1ms and the ultrasonic probe 600 at the detection position needs to emit the ultrasonic waves to the target body 62 from the angles of -10 degrees, 35 degrees, and 65 degrees, each array element 611 in the array set 61 is controlled to emit the ultrasonic wave towards the target body 62 according to the first ultrasonic emission sequence corresponding to -10 degrees, thereby achieving the emission of the ultrasonic wave from -10 degrees to the target body; after an interval of 1ms, each array element 611 in the array element set 61 is controlled to emit the ultrasonic wave to the target body 62 according to the second ultrasonic emission sequence corresponding to 35 degrees; after another interval of 1ms, each array element 611 in the array element set 61 is controlled to emit the ultrasonic wave to the target body 62 according to the third ultrasonic emission sequence corresponding to 65 degrees. In this way, it is realized that the ultrasonic waves are emitted from a plurality of angles to the target body 62 through one array element set 61. It should be noted that the time interval between the emissions of ultrasonic waves from two angles can be calculated starting from the time when the first array element in the array element set emits the ultrasonic wave, or starting from the time when the last array element in the array element set emits the ultrasonic wave, which is determined according to the specific requirements.

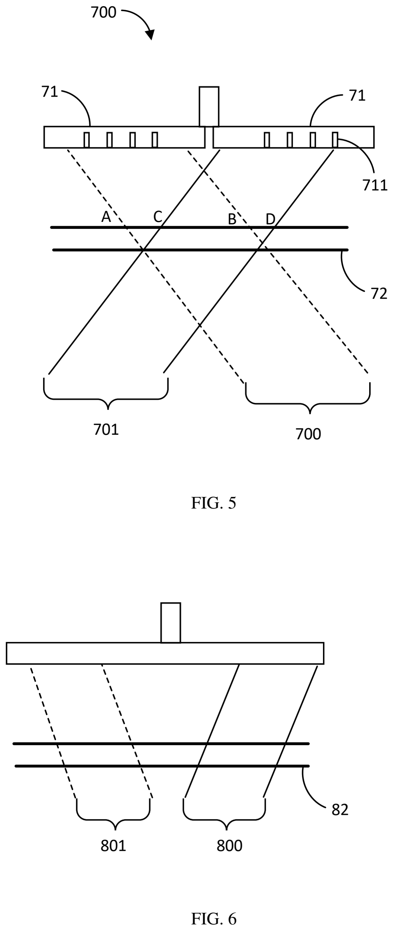

Please refer to FIG. 5, which is a schematic diagram of an ultrasonic probe 700 emitting ultrasonic waves provided by an embodiment of the present application. In FIG. 5, the ultrasonic probe 700 includes two array element sets 71. Each array element set 71 includes a plurality of array elements 711. In this case, when emitting the ultrasonic waves from a plurality of angles to the target body 72, the ultrasonic emission sequence corresponding to each angle can be firstly determined, and based on an angle emission timing sequence of each angle, array elements 711 in each array element set 71 are controlled sequentially to emit the ultrasonic waves to the target body 72 according to the ultrasonic emission sequences corresponding to respective angles. Where the angle emission timing sequence is used to characterize a sequence of ultrasonic waves emitted from each angle to the target body. That is, there will be a timing sequence for emitting the ultrasonic waves between various angles. Simply put, the plurality of array element sets 71 can work simultaneously, each array element set 71 emits the ultrasonic waves from one of the various angles to the target body 72, and different array element sets 71 emit the ultrasonic waves from different angles to the target body 72. In this way, the ultrasonic waves can be emitted simultaneously from a plurality of angles to the target body 72.

For example, assuming the ultrasonic probe 700 shown in FIG. 5 needs to emit ultrasonic waves to the target body 72 from the angles of -65 degrees and 65 degrees, where the angle emission timing sequence of -65 degrees is 1 and the angle emission timing sequence of 65 degrees is 2, then the left and right array element sets 71 can be controlled to work simultaneously, and each array element 711 in the left array element set 71 can be controlled to emit the ultrasonic wave to the target body 72 according to the first ultrasonic emission sequence corresponding to 65 degrees, and each array element 711 in the right array element set 71 can be controlled to emit the ultrasonic wave to the target body 72 according to the second ultrasonic emission sequence corresponding to -65 degrees. In this way, the ultrasonic waves can be emitted simultaneously from a plurality of angles to the target body 72.

It should be noted that the angle emission timing sequence of each angle can be set according to the requirements.

It can be understood that for at least some of the array element sets 71 in FIG. 5, it is also possible to control the array elements 711 in the array element set 71 to emit the ultrasonic waves to the target body 72 in the ultrasonic emission sequence corresponding to each angle at intervals of a preset time period. For example, based on the ultrasonic probe 700 shown in FIG. 5, it is needed to emit the ultrasonic waves to the target body 72 from -10 degrees, -20 degrees, 10 degrees, and 20 degrees, where the angle emission timing sequence is 10 degrees, -10 degrees, 20 degrees, and -20 degrees, respectively. So, each array element 711 in the left array element set 71 can be controlled to emit the ultrasonic waves to the target body 72 according to the first ultrasonic emission sequence corresponding to 10 degrees, and each array element 711 in the right array element set 71 can be controlled to emit the ultrasonic waves to the target body 72 according to the second ultrasonic emission sequence corresponding to -10 degrees; after a preset time period, each array element 711 in the left array element set 71 can be controlled to emit the ultrasonic waves to the target body 72 according to the third ultrasonic emission sequence corresponding to 20 degrees, and each array element 711 in the right array element set 71 can be controlled to emit the ultrasonic waves to the target body 72 according to the fourth ultrasonic emission sequence corresponding to -20 degrees.

It should be noted that in a case where the ultrasonic probe includes a plurality of array element sets, the present application does not limit the number of array element sets.

In the present application, for ultrasonic waves emitted from a plurality of different angles to the target body, these ultrasonic waves need to have an overlapping area in the imaging area of the target body. For example, please refer to FIG. 6, which is a schematic diagram of ultrasonic detection in some blood supply information extraction scenarios. In FIG. 6, the first ultrasonic wave 800 emitted from the first angle and the second ultrasonic wave 801 emitted from the second angle have no overlapping area in the imaging area of the target body 82. Therefore, these two ultrasonic waves do not meet the requirements.

In some embodiments, it is considered that the occurrence and development processes of many diseases are related to changes in the morphology or hemodynamics of microvessels. However, in the existing related technologies, the imaging of microvessels is performed based on the ultrasonic echo data of the narrow-beam ultrasonic waves. In this way, the data collection volume is relatively small, and the sensitivity of vascular imaging is low, and it is almost impossible to image microvessels with small diameters or extremely low blood flow velocities. In view of this, compared with the narrow-beam ultrasonic waves in some technologies, the beam width of at least some of the ultrasonic waves emitted from the plurality of angles in this present application may be not less than 3 ultrasonic wavelengths (i.e., at least some of the ultrasonic waves may be wide-beam ultrasonic waves). In this way, the frame rate can be increased, which in turn can increase the data collection volume. Furthermore, the microvessels can be imaged based on a larger data collection volume to extract the blood supply information of the microvessels. Herein, the frame rate can be the number of imaging operations that can be completed within a unit time T. The following comparison between FIG. 1 and FIG. 3 illustrates how the present application achieves the purpose of increasing data collection volume.

In FIG. 1, the ultrasonic probe 11 needs to emit the ultrasonic waves three times to perform one full data collection (complete one imaging operation) for the tissue 12, therefore, the frame rate is $\dfrac{T}{3t}$. Where T is the unit time, and t is the time taken to emit the ultrasonic wave once.

In FIG. 3, since the array elements 311 in the array element set 31 are controlled to emit the ultrasonic waves in different emission sequences, thus, wide-beam ultrasonic waves can be emitted by the array element set 31 by setting the width of the array element set 31 to be not less than 3 ultrasonic wavelengths. As the beam width of ultrasonic waves is relatively wide, the imaging area in the target body 32 is relatively large. The ultrasonic probe 31 may image the overlapping area CB once each time it emits an ultrasonic wave, so the frame rate is $\dfrac{T}{t}$.

By comparison, it can be seen that using the wide-beam ultrasonic waves can effectively improve the frame rate. As the number of the imaging operations for the overlapping areas within the unit time increases, more data information within the overlapping area can be obtained within the unit time. In turn, the microvessels can be further imaged based on this data information to extract the blood supply information of the microvessels.

Step S22, obtaining the blood supply information of the target body at each angle based on the ultrasonic echo data at each angle.

For ease of understanding, the extraction of the blood flow direction is taken as an example. Please refer to FIG. 7, which is a schematic diagram of a blood flow direction extraction provided by some embodiments of the present application. In FIG. 7, the exemplary array element set 91 emits a first ultrasonic wave 900 to the target body 92 from a first angle, and emits a second ultrasonic wave 901 to the target body 92 from a second angle (i.e., emits ultrasonic waves to the target body 92 from two different angles). The blood flow direction f1 of the target body 92 at the first angle can be extracted based on the first ultrasonic echo data of the first ultrasonic wave 900; the blood flow direction f2 of the target body 92 at the second angle can be extracted based on the second ultrasonic echo data of the second ultrasonic wave 901.

In some technologies, when extracting the blood supply information, the Butterworth high-pass filter is usually used to filter the echo data of the ultrasonic wave. However, this method has low accuracy, and even if the data volume of echo data is sufficient, it is still unable to extract the blood supply information of the microvessels. In view of this, in some embodiments of the present application, the ultrasonic echo data at each angle can be decomposed based on a principal component decomposition method to obtain the blood supply information at each angle. The principal component decomposition method has high accuracy, therefore, it can effectively identify microvessels in the overlapping area and extract the blood supply information of microvessels.

In some embodiments of the present application, the ultrasonic echo data at each angle is decomposed based on the principal component decomposition method, which can identify microvessels with blood flow velocities of less than 20 cm/s in the overlapping area and obtain the blood supply information of microvessels.

Optionally, in order to further improve the extraction efficiency of the blood supply information, the ultrasonic echo data at each angle can be decomposed in a parallel decomposition manner based on the principal component decomposition method to obtain the blood supply information at each angle. The decomposition efficiency of the ultrasonic echo data at each angle can be improved in a parallel processing manner, thus the extraction efficiency of the blood supply information at different angles can be improved.

In some embodiments, decomposing the ultrasonic echo data at each angle based on the principal component decomposition method to obtain the blood supply information at each angle includes:

storing the ultrasonic echo data at each angle in different matrices respectively to obtain an echo data matrix of each angle, and performing at least one row-column transposition operation on the ultrasonic echo data in the echo data matrix of each angle to decompose and obtain the blood supply information at each angle from the ultrasonic echo data after performing the row-column transposition operation.

Where, each ultrasonic echo data after performing the row-column transposition operation refers to the ultrasonic echo data in the echo data matrix of each angle after performing the row-column transposition operation.

Where, decomposing and obtaining the blood supply information at each angle from the ultrasonic echo data after performing the row-column transposition operation specifically can be: decomposing each ultrasonic echo data after performing the row-column transposition operation to obtain a decomposition result corresponding to each angle. Based on the decomposition result corresponding to each angle, the blood supply information at the corresponding angle can be obtained.

For ease of understanding, the following example provides an echo data matrix for one of the angles:

|     | a1  | a2  | a3  |
| --- | --- | --- | --- |
| t1  | A11 | A12 | A13 |
| t2  | A21 | A22 | A23 |
| t3  | A31 | A32 | A33 |
| ⋮   | ⋮   | ⋮   | ⋮   |
| tn  | An1 | An2 | An3 |

In the above echo data matrix, a1, a2, a3 can represent array elements, and t1 to tn can represent sampling points. Taking A11 as an example. A11 represents a first echo data corresponding to the array element a1 at the sampling point t1.

In the present application, since the echo data is obtained based on the wide-beam ultrasonic wave the frame rate is relatively high, resulting in a large amount of acquired data. On the other hand, since there are usually more sampling points (such as 3000) and fewer array elements, the number of rows in the echo data matrix of each angle may be much larger than the number of columns. In these two cases, if the data in the echo data matrix is directly decomposed, it will greatly increase the time consumption in the principal component decomposition process.

In view of this, in an embodiment of the present application, at least one row -column transposition operation is performed on the ultrasonic echo data in each echo data matrix during the decomposition process, and the blood supply information at each angle is decomposed and obtained from the ultrasonic echo data in each transposed echo data matrix. In this way, the purposes of reducing decomposition time and data processing volume are achieved.

Referring to FIG. 8, which is a schematic flowchart of principal component decomposition provided by an embodiment of the present application. In FIG. 8, the data decomposition process is carried out in the direction of the arrow. During decomposition, firstly, data is inputted (i.e., the ultrasonic echo data at each angle is respectively store in different matrices), and then the row-column transposition operation is performed so that the number of rows of the input data is less than the number of columns. In this way, when performing QR decomposition, it can avoid the problem of requiring excessive loops caused by the dependency relationship of data calculation, and thus greatly reduce the time consumption of parallel computing.

It can be understood that, in the result after QR decomposition, the number of columns is greater than the number of rows, which will increase the data processing volume in the subsequent principal component decomposition process, namely the SVD decomposition of the jacobi rotation. Specifically, during each iteration of the one-sided Jacobi rotation algorithm, it is necessary to rotate any two columns of data in the echo data matrix, and the (number of columns - 1) cycles are required in total. Since the row-column transposition was performed in the previous QR decomposition process, the number of columns in the echo data matrix is greater than the number of rows, which leads to a large data processing volume and longer decomposition time when performing the SVD decomposition of the jacobi rotation. Therefore, before performing the SVD decomposition of the jacobi rotation, another row-column transposition operation can be performed to make the number of columns smaller than the number of rows. In this way, during each iteration of the one-sided jacobi rotation algorithm, when rotating any two columns of data, due to the small number of columns, it can effectively reduce the data processing volume.

In the process of the principal component decomposition, before performing the QR decomposition, dimensionality reduction and power iteration operations can also be performed on the data after the row-column transposition.

Step S23, obtaining blood supply information of the target body in the overlapping area based on the blood supply information at each angle.

Specifically, since the overlapping area includes the imaging areas of the ultrasonic waves from various angles, the blood supply information corresponding to the overlapping area from each angle can be obtained based on the ultrasonic echo data of each angle. The blood supply information of each angle is integrated, so as to obtain the blood supply information of the target body in the overlapping area.

Continuing with FIG. 7 as an example, as mentioned earlier, the blood flow direction f1 of the overlapping area at the first angle can be extracted based on the first ultrasonic echo data of the first ultrasonic wave 900; the blood flow direction f2 of the overlapping area at the second angle can be extracted based on the second ultrasonic echo data of the second ultrasonic wave 901. The blood flow direction f 1 at the first angle and the blood flow direction f2 at the second angle are synthesized, so as to obtain the blood flow direction F of the overlapping area. In the present application, blood flow directions at a plurality of angles are synthesized to obtain the blood flow direction that can accurately reflect the blood flow direction in the overlapping area. Simply put, in some technologies (such as the scheme shown in FIG. 1), only the component of the blood flow direction of the target body in one direction can be extracted, which is not the actual blood flow direction of the target body. Whereas, in the present application, blood flow components at a plurality of angles are synthesized to obtain the true blood flow direction of the overlapping area. Therefore, the accuracy is high.

In summary, in technical solutions of some embodiments of the present application, the ultrasonic probe is controlled to emit ultrasonic waves from a plurality of different angles to the target body, and the imaging areas of the ultrasonic waves from the different angles in the target body have an overlapping area. In this way, there are ultrasound echo data at a plurality of angles in the overlapping area. Based on the ultrasonic echo data from a plurality of different angles, more accurate analysis can be performed on the blood supply information in the overlapping area, thereby, improving the accuracy of the blood supply information obtained in the overlapping area.

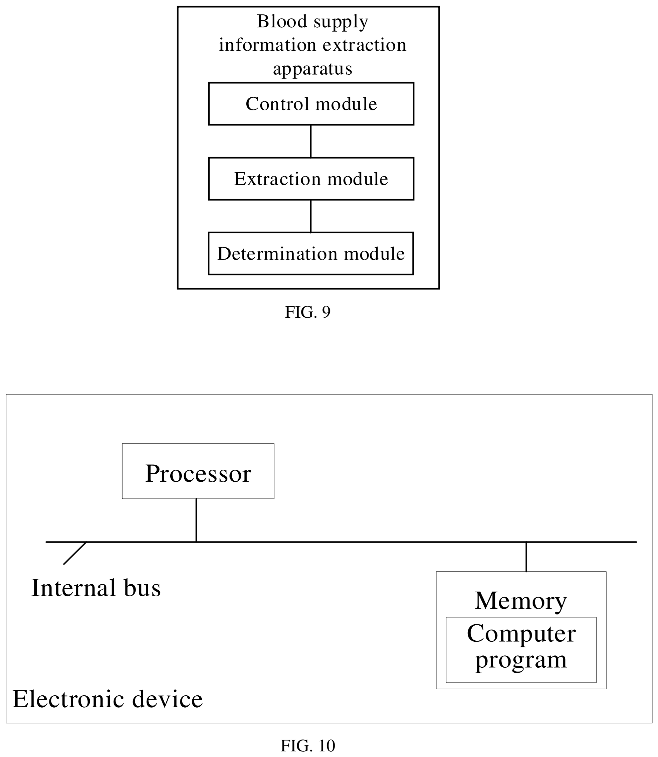

Please refer to FIG. 9, which is a schematic diagram of functional modules of a blood supply information extraction apparatus provided by an embodiment of the present application. The blood supply information extraction apparatus includes:

a control module, configured to control an ultrasonic probe to emit ultrasonic waves to a target body from a plurality of angles, and obtain ultrasonic echo data of the target body at the plurality of angles, where the ultrasonic wave emitted from each angle has a corresponding imaging area in the target body, and the imaging areas of the ultrasonic waves emitted from the plurality of angles in the target body have an overlapping area;

an extraction module, configured to, for the ultrasonic echo data at each angle, respectively perform a blood supply information extraction to obtain blood supply information of the target body at each angle; and

a determination module, configured to obtain blood supply information of the target body in the overlapping area based on the blood supply information at each angle.

Please refer to FIG. 10, which is a schematic structural diagram of an electronic device provided by an embodiment of the present application. The electronic device includes a processor and a memory, the memory is configured to store a computer program, which, when executed by the processor, implements the blood supply information extraction method as described above.

The processor can be a central processing unit (Central Processing Unit, CPU). The processor can also be other general-purpose processors, a digital signal processor (Digital Signal Processor, DSP), an application specific integrated circuit (Application Specific Integrated Circuit, ASIC), a field-programmable gate array (Field-Programmable Gate Array, FPGA), or other programmable logic devices, a discrete gate or a transistor logic device, a discrete hardware component, or a combination of the above types of chips.

The memory, as a non-transient computer-readable storage medium, can be configured to store non-transient software programs, non-transient computer executable programs, and modules, such as program instructions/modules corresponding to the methods in the embodiments of the present application. The processor executes various functional applications of the processor and data processing through running non-transient software programs, instructions, and modules stored in memory, that is, achieving the methods described in the above embodiments.

The memory may include a program storage area and a data storage area, where the program storage area may store an operating system and an application program required for at least one function; the data storage area can store data created by the processor, etc. In addition, the memory may include a high-speed random access memory, as well as a non-transient memory such as at least one disk storage device, a flash memory device, or other non-transient solid-state storage device. In some implementations, the memory may optionally include memories remotely disposed relative to the processor, and these memories can be connected to the processor via a network. Examples of the above networks include but are not limited to the Internet, enterprise intranet, local area network, mobile communication network and combinations thereof.

One embodiment of the present application also provides a computer-readable storage medium, and the computer-readable storage medium is used to store a computer program, which, when executed by a processor, implements the blood supply information extraction method described above.

One embodiment of the present application also provides a chip including a memory and a processor, where the memory stores code and data, the memory is coupled to the processor, and the processor runs a program in the memory to enable the chip to perform the blood supply information extraction method provided in any of the above embodiments.

One embodiment of the present application also provides a program product including a computer program, which, when the program product runs on a computer, enables the computer to perform the blood supply information extraction method provided in any of the above embodiments.

One embodiment of the present application also provides a computer program, which, when executed by a processor, performs the blood supply information extraction method provided in any of the above embodiments.

Although the embodiments of the present application have been described in conjunction with the accompanying drawings, those skilled in the art may make various modifications and variations without departing from the spirit and scope of the present application, and such modifications and variations fall within the scope defined by the appended claims.

**Claims**

1. A blood supply information extraction method, wherein the method comprises:

   controlling an ultrasonic probe to emit ultrasonic waves to a target body from a plurality of angles, and obtaining ultrasonic echo data of the target body at the plurality of angles, wherein the ultrasonic wave emitted from each angle has a corresponding imaging area in the target body, and the imaging areas of the ultrasonic waves emitted from the plurality of angles in the target body have an overlapping area;
   for the ultrasonic echo data at each angle, respectively performing a blood supply information extraction to obtain blood supply information of the target body at the each angle; and obtaining blood supply information of the target body in the overlapping area based on the blood supply information at the each angle.

2. The method according to claim 1, wherein a beam width of at least some of the ultrasonic waves emitted from the plurality of angles is not less than 3 ultrasonic wavelengths.

3. The method according to claim 1 or 2, wherein the ultrasonic probe comprises an array element set, and the array element set comprises a plurality of array elements;
   the controlling the ultrasonic probe to emit ultrasonic waves to the target body from the plurality of angles comprises:

   determining an ultrasonic emission sequence corresponding to each angle, wherein the ultrasonic emission sequence is used to characterize a time interval between emissions of the ultrasonic waves by each array element in the array element set;
   controlling each array element in the array element set to emit the ultrasonic wave to the target body according to the ultrasonic emission sequence corresponding to the each angle.

4. The method according to claim 3, wherein the controlling each array element in the array element set to emit the ultrasonic wave to the target body according to the ultrasonic emission sequence corresponding to the each angle comprises:
   controlling the array elements in the array element set to emit the ultrasonic waves to the target body according to the ultrasonic emission sequence corresponding to the each angle at intervals of a preset time period.

5. The method according to any one of claims 1 to 4, wherein the ultrasonic probe comprises a plurality of array element sets, each of the array element sets comprises a plurality of array elements;
   the controlling the ultrasonic probe to emit ultrasonic waves to the target body from the plurality of angles comprises:

   determining an ultrasonic emission sequence corresponding to the each angle, wherein the ultrasonic emission sequence is used to characterize a time interval between emissions of the ultrasonic waves by each array element in the array element set;
   sequentially controlling the array elements of each array element set to emit the ultrasonic waves to the target body according to the ultrasonic emission sequences corresponding to the respective angles based on an angle emission timing sequence of the plurality of angles.

6. The method according to any one of claims 1 to 5, wherein for the ultrasonic echo data at each angle, the respectively performing the blood supply information extraction to obtain the blood supply information of the target body at the each angle comprises:
   performing decomposition on the ultrasonic echo data at the each angle based on a principal component decomposition method to obtain the blood supply information at the each angle.

7. The method according to claim 6, wherein the performing decomposition on the ultrasonic echo data at the each angle based on the principal component decomposition method to obtain the blood supply information at the each angle comprises:
   storing the ultrasonic echo data at the each angle in different matrices respectively to obtain an echo data matrix of the each angle, and performing at least one row-column transposition operation on the ultrasonic echo data in the echo data matrix of the each angle to decompose and obtain the blood supply information at the each angle from each ultrasonic echo data after performing the row-column transposition operation.

8. The method according to claim 6 or 7, wherein the performing decomposition on the ultrasonic echo data at the each angle comprises:
performing decomposition on the ultrasonic echo data at the each angle using a parallel decomposition manner.

9. The method according to any one of claims 6 to 8, wherein the performing decomposition on the ultrasonic echo data at the each angle based on the principal component decomposition method to obtain the blood supply information at the each angle comprises:
performing parallel decomposition on the ultrasonic echo data at the each angle using a graphics processor based on a principal component decomposition method to obtain the blood supply information at the each angle.

10. A blood supply information extraction apparatus, wherein the apparatus comprises:

   a control module, configured to control an ultrasonic probe to emit ultrasonic waves to a target body from a plurality of angles, and obtain ultrasonic echo data of the target body at the plurality of angles, wherein the ultrasonic wave emitted from each angle has a corresponding imaging area in the target body, and the imaging areas of the ultrasonic waves emitted from the plurality of angles in the target body have an overlapping area;
   an extraction module, configured to, for the ultrasonic echo data at each angle, respectively perform a blood supply information extraction to obtain blood supply information of the target body at the each angle;
   a determination module, configured to obtain blood supply information of the target body in the overlapping area based on the blood supply information at the each angle.

11. A computer-readable storage medium, wherein the computer-readable storage medium is used to store a computer program, which, when executed by a processor, implements the method according to any one of claims 1 to 9.

12. An electronic device, wherein the electronic device comprises a processor and a memory, the memory is configured to store a computer program, which, when executed by the processor, implements the method according to any one of claims 1 to 9.

13. A chip, wherein the chip comprises a memory and a processor, the memory stores code and data, the memory is coupled to the processor, and the processor runs a program in the memory to enable the chip to perform the method according to any one of claims 1 to 9.

14. A program product, wherein it comprises a computer program, which, when the program product runs on a computer, enables the computer to perform the method according to any one of claims 1 to 9.

15. A computer program, wherein when executed by a processor, the computer program is configured to perform the method according to any one of claims 1 to 9.

FIG. 1

FIG. 2

300

31

311

A

θ
α

B

32

D

C

FIG. 3

600

61

611

A    C         B         D

62

601                              600

FIG. 4

700

71                                    71

711

A   C       B   D

72

701            700

FIG. 5

82

801    800

FIG. 6

91

f2

A    C    B    D

F

f1

92

901                    900

FIG. 7

Input
data

Transpo-
sition

Data a

Dimensi-
onality
reduction

Data b

Power
iteration

Data c

Decomposition

Data f

Decomposition

Data e

Transpo-
sition

Data d

FIG. 8

Blood supply
information extraction
apparatus

Control module

Extraction module

Determination module

FIG. 9

Processor

Internal bus

Memory

Computer
program

Electronic device

FIG. 10

## INTERNATIONAL SEARCH REPORT

| International application No. |
| --- |
| **PCT/CN2024/096056** |

**A. CLASSIFICATION OF SUBJECT MATTER**

A61B8/06(2006.01)i;  A61B8/00(2006.01)i

According to International Patent Classification (IPC) or to both national classification and IPC

**B. FIELDS SEARCHED**

Minimum documentation searched (classification system followed by classification symbols)

IPC:A61B8/-

Documentation searched other than minimum documentation to the extent that such documents are included in the fields searched

Electronic data base consulted during the international search (name of data base and, where practicable, search terms used)

CNTXT: CNKI; TWTXT; USTXT; ENTXTC; DWPI: 无锡海斯凯尔医学技术有限公司, 何琼, 许晓臣, 邵金华, 孙锦, 深圳迈瑞, 血流, 叠加, 重叠, 覆盖, 交集, 角度, 时间, 间隔, 向量, ultrasonic, blood flow, overlap, angle, direction, interval, vector

**C. DOCUMENTS CONSIDERED TO BE RELEVANT**

| Category* | Citation of document, with indication, where appropriate, of the relevant passages | Relevant to claim No. |
| --- | --- | --- |
| PX | CN 116671980 A (WUXI HISKY MEDICAL TECHNOLOGIES CO., LTD.) 01 September 2023 (2023-09-01) <br> description, paragraphs 47-100, and figures 1-10 | 1-15 |
| X | US 2021378626 A1 (SHENZHEN MINDRAY BIO-MEDICAL ELECTRONICS CO., LTD.) 09 December 2021 (2021-12-09) <br> claim 1, description, paragraphs 47-111, and figures 6-13b | 1-15 |
| X | CN 113662585 A (SHENZHEN MINDRAY BIO-MEDICAL ELECTRONICS CO., LTD.) 19 November 2021 (2021-11-19) <br> description, paragraphs 52-124 | 1-15 |
| A | CN 105310726 A (TOSHIBA K.K. et al.) 10 February 2016 (2016-02-10) <br> entire document | 1-15 |
| A | CN 106456118 A (VERSITECH LTD.) 22 February 2017 (2017-02-22) <br> entire document | 1-15 |
| A | CN 108135579 A (HITACHI LTD.) 08 June 2018 (2018-06-08) <br> entire document | 1-15 |

☑ Further documents are listed in the continuation of Box C. ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **29 August 2024** | **04 September 2024** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/096056** |

| C. | DOCUMENTS CONSIDERED TO BE RELEVANT | |
| --- | --- | --- |
| Category* | Citation of document, with indication, where appropriate, of the relevant passages | Relevant to claim No. |
| A | CN 109414245 A (SHENZHEN MINDRAY BIO-MEDICAL ELECTRONICS CO., LTD.) 01 March 2019 (2019-03-01)<br>entire document | 1-15 |
| A | CN 111093515 A (SHENZHEN MINDRAY BIO-MEDICAL ELECTRONICS CO., LTD.) 01 May 2020 (2020-05-01)<br>entire document | 1-15 |

Form PCT/ISA/210 (second sheet) (July 2022)

## INTERNATIONAL SEARCH REPORT
### Information on patent family members

International application No.

**PCT/CN2024/096056**

| Patent document cited in search report | | | Publication date (day/month/year) | Patent family member(s) | | | Publication date (day/month/year) |
|---|---|---|---|---|---|---|---|
| CN | 116671980 | A | 01 September 2023 | None | | | |
| US | 2021378626 | A1 | 09 December 2021 | WO | 2020124349 | A1 | 25 June 2020 |
| CN | 113662585 | A | 19 November 2021 | None | | | |
| CN | 105310726 | A | 10 February 2016 | US | 2015366540 | A1 | 24 December 2015 |
| | | | | US | 10603014 | B2 | 31 March 2020 |
| | | | | JP | 2016002379 | A | 12 January 2016 |
| | | | | JP | 6282942 | B2 | 21 February 2018 |
| CN | 106456118 | A | 22 February 2017 | US | 2017215836 | A1 | 03 August 2017 |
| | | | | US | 10335113 | B2 | 02 July 2019 |
| | | | | US | 2019320998 | A1 | 24 October 2019 |
| | | | | US | 11154272 | B2 | 26 October 2021 |
| | | | | US | 2015141832 | A1 | 21 May 2015 |
| | | | | US | 10231695 | B2 | 19 March 2019 |
| | | | | EP | 3195806 | A1 | 26 July 2017 |
| | | | | EP | 3195806 | B1 | 06 April 2022 |
| | | | | WO | 2015074511 | A1 | 28 May 2015 |
| | | | | EP | 3071111 | A1 | 28 September 2016 |
| | | | | EP | 3071111 | B1 | 09 March 2022 |
| CN | 108135579 | A | 08 June 2018 | JPWO | 2017068892 | A1 | 26 July 2018 |
| | | | | JP | 6457107 | B2 | 23 January 2019 |
| | | | | WO | 2017068892 | A1 | 27 April 2017 |
| CN | 109414245 | A | 01 March 2019 | WO | 2018058606 | A1 | 05 April 2018 |
| CN | 111093515 | A | 01 May 2020 | US | 2021007706 | A1 | 14 January 2021 |
| | | | | US | 11602324 | B2 | 14 March 2023 |
| | | | | WO | 2019183990 | A1 | 03 October 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310620741